# EUROPEAN PATENT SPECIFICATION

(11) **EP 2 051 991 B1**
(45) Date of publication and mention of the grant of the patent: **12.10.2011**
(21) Application number: 07794118.5
(22) Date of filing: 17.08.2007
(51) Int. Cl.: C07K 5/10, C07K 5/107, A61K 38/07, A61P 25/04, A61P 9/10

(54) **CARDIOPROTECTIVE COMPOUNDS**
CARDIOPROTEKTIVE VERBINDUNGEN
COMPOSÉS CARDIOPROTECTEURS

(30) Priority: 17.08.2006 SE 0601695
(43) Date of publication of application: 29.04.2009
(73) Proprietor: Eribis Pharmaceuticals AB, 754 50 Uppsala (SE)
(72) Inventor: BOBROVA, Irina, S-723 53 Västerås (SE)
(74) Representative: VALEA AB
(86) International application number: PCT/SE2007/000731
(87) International publication number: WO 2008/020802

(56) References cited:
- EP-A- 0 127 154
- JP-A- 55 141 453
- BOBROVA ET AL: "Synthesis and biological activity of branched enkephalin analogues" EUROPEAN JOURNAL OF MEDICINAL CHEMISTRY, EDITIONS SCIENTIFIQUE ELSEVIER, PARIS, FR, vol. 33, no. 4, April 1998 (1998-04), pages 255-266, XP005276605 ISSN: 0223-5234
- BOBROVA ET AL: "Synthesis and biological activity of branched enkephalin analogues containing two amino acids in a side chain" EUROPEAN JOURNAL OF MEDICINAL CHEMISTRY, EDITIONS SCIENTIFIQUE ELSEVIER, PARIS, FR, vol. 33, no. 4, April 1998 (1998-04), pages 331-334, XP005276611 ISSN: 0223-5234
- N M INKINA AND G F ROSENTAL: "Investigation of the Receptor Selectivity Profile of Short Enkephalin Analogs Bearing D-ornithine at the Second Position of a Molecule" UKRAINSKII BIOKHIMICHESKII ZHURNAL, vol. 66, no. 2, 1994, pages 64-70, XP008085654
- JO EL J SCHULTZ, GARRETT J GROSS: "Opioids and cardioprotection" PHARMACOLOGY AND THERAPEUTICS, vol. 89, February 2001 (2001-02), pages 123-137, XP002457805
- ZAKUSOV V V ET AL: "EFFECT OF AGONISTS AND ANTAGONISTS OF OPIATE RECEPTORS ON ANIMAL RESISTANCE TO HYPOXIC HYPOXIA" BYULLETEN EKSPERIMENTALNOI BIOLOGII I MEDITSINY, MEDICINA, MOSCOW, SU, vol. 98, no. 12, 1984, pages 680-682, XP008085613 ISSN: 0365-9615
- JEAN-LUC FAUCHERE ET AL.: "Interaction of p-Nitrophenylalanine Enkephalins with mu- delta- and kappa-Subtypes of the Opiate Receptor" EUROPEAN JOURNAL OF PHARMACOLOGY, vol. 77, 5 February 1982 (1982-02-05), pages 339-342, XP002457804
- YU B LISHMANOV ET AL: "Activation of [delta]-opioid receptors increases resistance of isolated heart to ischemia/reperfusion: The role of cAMP and intracellular calcium" BIOLOGY BULLETIN OF THE RUSSIAN ACADEMY OF SCIENCES, KLUWER ACADEMIC PUBLISHERS-PLENUM PUBLISHERS, NE, vol. 32, no. 1, 1 January 2005 (2005-01-01), pages 45-51, XP019294189 ISSN: 1608-3059
- RICHARD L ELLIOT ET AL.: "Novel Asp32-Replacement Tetrapeptide Analogues as Potent and Selective CCK-A Agonists" JOURNAL OF MEDICINAL CHEMISTRY, vol. 37, 27 May 1994 (1994-05-27), pages 1562-1568, XP002457806
- HARDY G W ET AL: "PERIPHERALLY ACTING ENKEPHALIN ANALOGUES POLAR TRI- AND TETRAPEPTIDES" JOURNAL OF MEDICINAL CHEMISTRY, AMERICAN CHEMICAL SOCIETY. WASHINGTON, US, vol. 32, no. 5, 1 May 1989 (1989-05-01), pages 1108-1118, XP000611482 ISSN: 0022-2623

## Description

The present invention relates to peptides and peptide-based compounds which are useful as tissue protective compounds, for example cardioprotective compounds.

Methionine-enkephalin and leucine-enkephalin are pentapeptides with the respective structures Tyr-Gly-Gly-Phe-Met and Tyr-Gly-Gly-Phe-Leu (Hughes J, Smith TW, Kosterlitz HW, Fothergill LA, Morgan BA, Morris HR (1975) Nature, Lond. 258, 577-579). Many investigations carried out on these and similar compounds have focused on analgesic activity.

For example, analgesic activity has been demonstrated for compounds based on the tetrapeptide Tyr-D-Orn-Gly-Phe wherein a Pro, Leu, Asn or Met residue is attached to the δ-amino group of D-ornithine (Bobrova et al: European Journal of Medicinal Chemistry (1998), 33(4), pages 255-266), and for Tyr-D-Orn-Gly-Phe(NO₂)-NH₂ wherein a dipeptide chain is attached to the δ-amino group of D-ornithine (Bobrova et al: European Journal of Medicinal Chemistry (1998), 33(4), pages 331-334).

In contrast there have been relatively few investigations into cytoprotection or tissue protection by enkephalin analogues.

Tyr-D-Ala-Gly-Phe(NO₂)-NH₂ has been shown to have an antihypoxic effect (Zakusov, V. V. et al: Byulleten Eksperimentalnoi Biologii i Meditsiny (1984), 98(12), pages 680-682).

Analogue [D-Ala2,MePhe4Met(O)5-ol]-enkephalin has shown protective effects against gastric damage (Ferri, S., Speroni, E., Candeletti, S., Cavicchini, E, Romualdi, P., Govoni, P-, & Marchini, M. (1988). Pharmacology 36, 140-144; Ferri, S., Arrigo-Reina, R., Candeletti, S., Costa, S., Murari, G., Speroni, E., & Scoto, G (1983), Pharmocol Res Commun 15, 409-418)

Dalargin [D-Ala2, Leu5, Arg6] has also shown protective effects against gastric damage (Timoshin SS, Shvets SI, Radivoz MI, Aleksandrovich AG, Mel'nik EI, Biull Eksp Biol Med. 1991 Aug;112(8):130-2).

[D-Ala2-D-Leu5]-enkephalin (DADLE), has been shown to be efficacious in extending multi-organ survival time and tissue preservation prior to organ transplantation (Chien, S., Oeltgen, P. R., Diana, J. N., Shi, X., Nilekani, S. P., & Salley, R. (1991) J Thorac Cardiovasc Surg 102, 224-234; Chien, S., Oeltgen, P. R., Diana, 1. N., & Salley, R. K. (1994) J Thorac Cardiovasc Surg 107, 964-967; Oeltgen, P. R., Horton, N. D., Bolling, S. F., & Su, T..P. (1996) Ann Thorac Surg 61, 1488-1492).

The role of delta-opioid receptor activation has been investigated. It has been found that [D-Pen(2,5)]enkephalin (DPDPE); deltorphin-D, a novel delta(2)-opioid agonist pretreatment reduced infarct size, whereas [D-Ala(2),D-Leu(5)]enkephalin (DADLE) had no effect (Am J Physiol Heart Circ Physiol. 2002 Jun;282(6):H1953-60). Takasaki et al. have demonstrated that both native enkephalins produced protection, whereas, β-endorphin had no such effect [Takasaki Y., Wolff R.A., Chien G.L.,Van Winkle D.M., 1999, Am.J.Physiol, 277, H2442-50] (a study in isolated rabbit myocytes).

There are many analgesic drugs available on the market, such that it is possible to select an appropriate one for a particular scenario. In contrast there is arguably a greater need for effective tissue-protective compounds, such as cardioprotective compounds. For example, the treatment of myocardial infarction remains a medical priority. We have now found new compounds which exhibit useful tissue protective effects.

From a first aspect the present invention provides a compound comprising the tetrapeptide structure **A-B-C-D,** or a pharmaceutically acceptable salt thereof.

The tetrapeptide structure **A-B-C-D** referred to herein is defined, according to convention, from its N-terminus to its C-terminus.

The N-terminus of the compound, which is on amino acid residue **A**, may be unsubstituted, i.e. may be a free NH₂ group. Alternatively each hydrogen of this NH₂ group may independently optionally be replaced by C₁₋₅ alkyl or C₁₋₅ acyl, wherein C₁₋₅ denotes a carbon chain of 1 to 5 carbon atoms that may be straight or branched, for example methyl, ethyl, n-propyl, iso-propyl, n-butyl, iso-butyl, tert-butyl, n-pentyl and isomers thereof.

**A** is a D or L-configured aromatic amino acid residue, preferably in the L-configuration. In some aspects of the invention the aromatic ring of this moiety may contain one or more nitro group. However, such a nitro group is not mandatory and effective results have been obtained without a nitro group in this position. Some suitable amino acids for **A** include Tyr, Thyronine, Phe or Trp. In some aspects of the invention, the aromatic ring contains a hydroxy substituent, preferably in the *p*-position. **A** is preferably Tyr.

**B** is a D- or L-configured diamino acid residue wherein the side chain amino group is substituted with C₁₋₅ acyl, C₁₋₅ alkyl, **-B'**-C₁₋₅ acyl or **-B'**-C₁₋₅ alkyl, wherein **B'** is an amino acid residue which may optionally be substituted with one or more nitro group. However, such a nitro group is not mandatory and effective results have been obtained without a nitro group in this position. Accordingly it is preferred that **B'** is not substituted with any nitro group.

**B** is preferably in the D-configuration.

Since **B** is a diamino acid residue, one amino group is used to form a peptide bond to moiety **A** and the other amino group is on the side chain and derivatized as described herein. Suitable diamino acid residues include Dap, Dab, Orn, Lys, 4,5-dehydro-lysine or 2,6-diamino-4-hexynoic acid, preferably Dab, Orn or Lys, more preferably Orn. In other aspects of the invention B may be Lys. In other aspects of the invention **B** may be Dab.

The side chain amino group of **B** is substituted with a C₁₋₅ acyl group or a C₁₋₅ alkyl group, resulting in either an amide linkage at this point or a secondary amine. This favourably affects the activity of the compound. Substitution with a C₁₋₅ acyl group is preferred. It is believed that this contributes to effectiveness by altering charge and polarity. A D- or L- configured (preferably L-configured) amino acid residue **B'** may be present between **B** and the C₁₋₅ acyl group or C₁₋₅ alkyl group, in which case the side chain amino group of **B** forms a peptide bond with the carbonyl group of **B'** and the amino group of **B'** is substituted with a C₁₋₅ alkyl group or (preferably) a C₁₋₅ acyl group.

Preferably the C₁₋₅ acyl group is acetyl.

Preferably **B'**, if present, is Gly, Ala, Pro, Leu, Asn or Met, more preferably Gly or Asn.

**C** is Gly wherein the peptide N-H may optionally be (though preferably is not) changed to N-methyl. In other words, the backbone may be modified such that the nitrogen atom of Gly may be methylated.

**D** is D- or L-Phe, preferably L-Phe, which is substituted on the aromatic ring with at least one nitro group. This nitro group enhances the activity of the compound. There may also be up to four other further substituents on the aromatic ring, independently selected from nitro, fluoro, chloro, bromo, iodo, trifluoromethyl or cyano. Only one nitro substituent is however essential. The nitro substituent is preferably in the para position. The peptide N-H may optionally be (though preferably is not) changed to N-methyl. In other words the backbone may be modified so that the nitrogen atom of Phe may be methylated.

The C-terminus at residue D can take the form of the free acid -COOH or alternatively -CONH₂, -CONHC₁₋₅ alkyl, -CONH-NH₂, -CONH-NHC₁₋₅ alkyl, COO-C₁₋₅ alkyl, or -CH₂OH. In some embodiments the C-terminus at residue D does not take the form of the free acid -COOH or -CH₂OH, but is selected from -CONH₂, -CONHC₁₋₅ alkyl, -CONH-NH₂, -CONH-NHC₁₋₅ alkyl, or COO-C₁₋₅ alkyl. Preferably it is -CONH₂. In combination with the other moieties this results in compounds with high activity *in vitro* and in *in vivo* biotests.

From further aspects the present invention provides pharmaceutical compositions comprising compounds of the invention and the compounds for use as medicaments.

From further aspects the present invention provides the use of these compounds for the manufacture of medicaments for tissue protective treatment or cardioprotective treatment.

Compounds of the invention protect tissue, for example heart muscle, which is subjected to ischemia. We have also observed pronounced antihypoxic effects and notable analgesic activity. The combination of these three effects is particularly advantageous in several areas of medicine including cardiology and intensive care. Control of the ischemic syndrome and circulatory hypoxia is of importance in several conditions.

Therefore, in further aspects compounds of the invention are additionally or alternatively of use in antihypoxic and/or analgesic treatment.

In some aspects the present invention relates to peptides and peptide-based compounds which are useful as antiischemic medicines, for myocardial ischemia (for example ischemic chest pain and myocardial infarction) or in order to ameliorate the effect of a particular brain malfunction, such as ischemic brain damage (stroke etc). Antihypoxic properties of our new compounds increase stability to oxygen-dependent pathological conditions, such as malfunction of lungs, liver, eyes, and pathology of delivery, shock, bleeding, and physical overloading. The analgesic effect of the new substances can be useful in alleviating the pain associated with, for example, a myocardial infarction.

In some aspects the new compounds exhibit significant useful cardioprotective, antihypoxic and analgesic effects. The pronounced analgesic effect can be useful in alleviating the pain associated with a myocardial infarction. Taken together, the combined effects are not previously known but are highly desirable.

A preferred compound according to the invention is:

Tyr-D-Orn(Ac)-Gly-Phe(p-NO₂)-NH₂ (1)

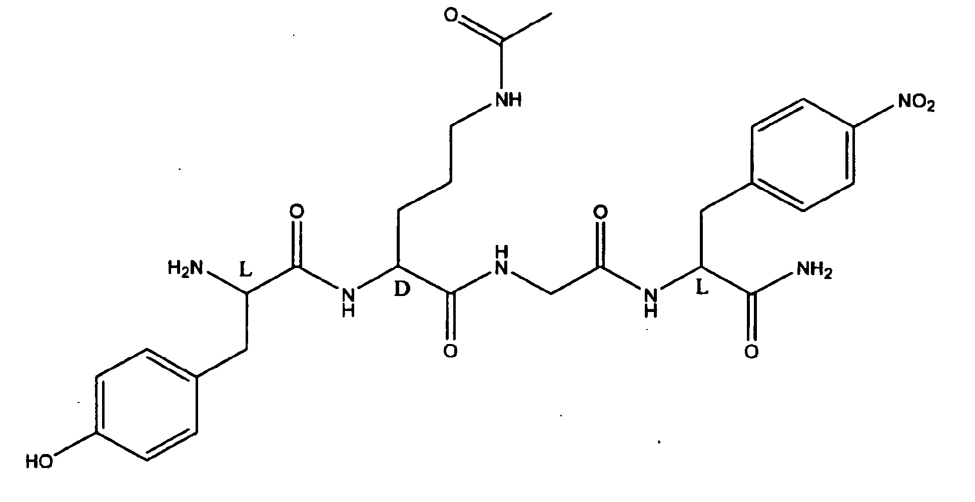

Four compounds (modification of the position **B** in the general formula **A-B-C-D**) according to the invention are:

Tyr-D-Lys(Ac)-Gly-Phe(p-NO₂)-NH₂ (2)

Tyr-D-Orn(Ac-Asn)-Gly-Phe(p-NO₂)-NH₂ (3)

Tyr-D-Dab(Ac)-Gly-Phe(p-NO₂)-NH₂ (4)

Tyr-D-Orn(Ac-Gly)-Gly-Phe(p-NO₂)-NH₂ (5)

### Brief description of the drawings

Figure 1. Illustration of experimental protocol for the study of the effect of peptides (1,2,3,4,5) in the rat model of myocardial infarction. Peptide is administered at - 5 min before occlusion. Infarct size is analysed after 120 min reperfusion period.
Figure 2. Peptide (4) decreased infarct size expressed as a percentage of the area-at risk (IS/AAR) when administered i.v. to male Sprague-Dawley rats 5 min before a 25 min occlusion and 120 min reperfusion period.

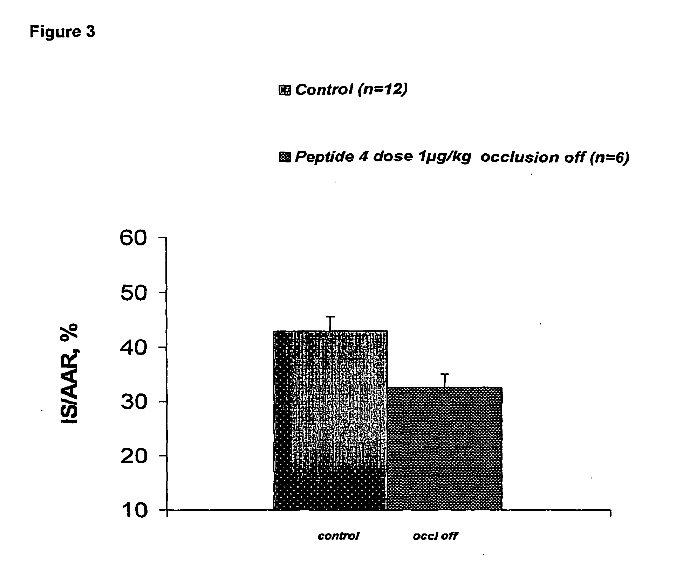

Figure 3. Peptide (4) decreased infarct size expressed as a percentage of the area-at risk (IS/AAR) when administered i.v. to male Sprague-Dawley rats immediately after removal of the 25 min Left Coronary Artery Occlusion period in comparison with control.
Figure 4. Analgesic Activity of Enkephalin Analogues (1-5) at intravenous administration to mice ("hot plate" method)
Figure 5. Potency of the effect of jump latency time, expressed as the area under a curve from the point of "5 minutes" till the point of 90 minutes"
Figure 6. Dose-response diagrams for jump latency time at the point of 15 minutes after the peptides (A) and morphine (B) administration

### Examples

### Abbreviations.

Symbols and abbreviations are in accord with the recommendations of the IUPAC-IUB Joint Commission on Biochemical Nomenclature (Biochem.J.(1984), 219, 345-374). All optically active amino acids have L-configuration unless otherwise stated. Abbreviations used: DCC, dicyclohexylcarbodiimide; DCU, dicyclohexyl urea; DMF, dimethyl-formamide; HOBt, 1-hydroxybenzotriazol; OPcp, pentachlorophenyl; OPfp, pentafluorophenyl; p-NO₂, para-nitro; RP-HPLC, reverse-phase high performance liquid chromatography; DIEA, diisopropylethylamine; TFA, trifluoroacetic acid; DCM, dichloromethane; MeOH, methanol; MeCN, acetonitrile; *n*BuOH *n*-butanol, *i*PrOH, isopropanol; NH₄OBt, 1-hydroxybenzotriazol ammonium salt; DPPA, diphenylphosphorylazide; i.cist., intracisternal; i.v., intravenous; i.p.,intraperitoneal; OR, opiate receptor; GPI, guinea pig ileum; MVD, mouse vas deferens; Phe(*p*-NO₂), 4-nitro-L-phenylalanine; Dab, α,γ-diaminobutyric acid; Orn, ornithine; Boc, tert-butyloxy-carbonyl; Z, benzyloxycarbonyl; MBHA resin, 4-methylbenzhydrylamine resin; Ac, acetyl; AcOH, acetic acid.

### Chemistry

Synthesis of the enkephalin analogue (peptide 1) was performed by the classical methods of peptide chemistry (in solution).

The analogues (**2,3,4,5**) were synthesized by solid-phase methodology using BOC/Bzl-chemistry and N,N'-diisopropylcarbodiimide as an coupling agent.

### Experimental protocols

### Example 1

### Synthesis of enkephalin analogue (1)

Synthesis of the enkephalin analogue (1) was performed using active esters and azide coupling methods. All amino acids have L-configuration, except D-ornithine.

Amino acid derivatives were received from Reanal (Hungary). Evaporation was performed in vacuo at 40°C. Melting points (uncorrected) were determined using a digital melting point analyzer "Fisher", model 355. ¹H-NMR spectra were recorded on Bruker WM-360 spectrometer.

The purity of synthesised compounds was substantiated by TLC on Silufol UV 254 (Czechoslovakia) or Silica gel 60F 254 received from Merck (Germany) in systems: A, CHCl₃-EtOH-AcOH (85:10:5); B, nBuOH-pyridine-AcOH-H₂O (15:10:3:6); C, nBuOH-AcOH-H₂O (4:1:1); D, CHCl₃-MeOH-AcOH-H₂O (30:20:4:6); E, (60:18:2:3). Amino acid analysis was performed in a Biocal BC-200 amino acid analyser after peptide hydrolysis in a sealed ampoule (24 hrs at 110°C).

Protected peptide was purified on chromatographic columns of "Merck": size C (440-37). The following solvent systems were used for evaluation: CHCl₃-EtOH-ethylacetate-AcOH-H₂O, 185:5:8:2:0.25 (chI); 85:5:8:2:0.25 (chII).

HPLC for analytical purposes was performed on a Dupont model 8800 HPLC system, using a column (4.6x110mm) packed with reverse-phase sorbent Silasorb C₁₈ (Lachema). Optical rotations were determined with a Perkin-Elmer141 polarimeter with the 10-cm water jacketed cell in solvents and at concentrations specified below.

### Boc-Gly Phe(p-NO₂)(6)

4.2 g (20 mmol) of L-Phe(p-NO₂) were dissolved in 20 ml of 1N NaOH solution, added 1.68 g of NaHCO₃, 25 ml of DMF and, at stirring and cooling down to 5°C, 8.47 g (20 mmol) of Boc-Gly-OPcp dissolved in 10 ml of DMF. After stirring during several hours, when the reaction was over (chromatographic control) the mixture was diluted with ethyl acetate and water and acidified with 5% KHSO₄ solution to pH 3. The ethyl acetate layer was separated and water phase was extracted with two portions of ethyl acetate additionally. The three organic phases were combined, washed with water, saturated water solution of NaCl and finally dried over anhydrous Na₂SO₄. After solvent evaporation the crystalline precipitate was re-crystallised from ethyl acetate with a small addition of petroleum ether. The yield was 4.67 g (63%) of dipeptide (**6**), m.p. 167-168°C, [a]_{D}²⁴+46.9° (c 1, EtOH), R_{f} 0.79 (A); 0,77 (B);0.83(E).

### Boc-Gly-Phe(p-NO₂) -NH₂(7)

1.52 g (10 mmol) of NH₄OBt and 2.06 g (10 mmol) of DCC were added to 3.67 g (10 mmol) of compound (**6**) dissolved in 20 ml of DMF. The reaction mixture was left for night. DCU was filtered and after solvent evaporation the residue was dissolved in chloroform. The organic phase was washed with 5% KHSO₄ solution, water, 5% NaHCO₃ solution, water, a saturated aqueous solution of NaCl and finally dried over anhydrous Na₂SO₄. After evaporation of the solvent the product was crystallised from absolute ethanol with small amount of ethylacetate. The yield was 2.72 g (74%) of protected dipeptide amide (**7**) m.p. 169-171°C, [a]_{D}²²-9.2° (c 1, DMF), R_{f} 0.84(A); 0,88 (B);0.66(E).

### Boc-Tyr(Boc)-D-Orn(Z)(9)

2.66 g (10 mmol) of D-Orn(Z) were dissolved in 10 ml of 1N NaOH solution, added 0.84 g of NaHCO₃, 25 ml of DMF and, at stirring and cooling down to -5°C, 5.47 g (10 mmol) of Boc-Tyr(Boc)-OPfp dissolved in 15 ml of DMF. The reaction was carried out for several hours and left for night. After the reaction was over, the material was treated similarly to compound (**6**). The solvent was removed and the product was crystallised from diethyl ether and n-hexane. The solid residue was re-crystallised from the same mixture of solvents and dried in vacuo. The yield was 3.28 g (52%) of protected dipeptide (**9**) m.p. 112-114°C, [a]_{D}²² +4.2°(c 0.5, EtOH), R_{f} 0.66(A); 0,52 (A, "Merck");0.78(B "Merck").

### Boc-Tyr(Boc)-(N⁵-Ac)-D-orn (11)

Palladium black was added to 2.1 g (3.3 mmol) of protected dipeptide **(9)** in 10ml of MeOH, 0.5ml of AcOH and 5ml of H₂O and hydrogenated during several hours. After the reaction was over (chromatographic control) the catalyst was filtered and the filtrate was evaporated. The residue was dissolved in ether with small amount of ethyl acetate. The mixture was left in refrigerator until all the product was crystallised out. The product was filtered, washed with cold ether on filter and dried *in vacuo*. The yield was 1.54 g (83%) of compound **(10)** with R_{f}0.16 (C "Merck"); 0.66 (B "Merck"); 0.67 (D).

8.32 g (15 mmol) of dipeptide acetate **(10)** were dissolved in 50ml of DMF and upon stirring and cooling 5.1 ml (30 mmol) of DIEA in 15 ml of DMF and 2.7 g (15 mmol) of p-nitrophenyl acetate dissolved in 5ml of DMF were added. The reaction mixture was stirred during 2-3 hrs and when the reaction was over (chromatographic control) was worked up similar to compound **(6)**. The solvent was removed and the residue was dissolved in ether and reprecipitated with hexane twice. The product was filtered and dried *in vacuo*. The yield was 6.52 g (81%) of protected dipeptide (11) with R_{f} 0.33 (A "Merck"); 0.55 (E "Merck"); m.p. 95°C, [a]_{D}²² -9.6°(c 0.5, EtOH),

### Doc-Tyr(Boc)-(N^{δ}-Ac)-D-Orn-Gly-Phe(p-NO₂) -NH₂(12)

4.75 g (13 mmol) of dipeptide (3) were dissolved in 30ml of 50% TFA in DCM and kept for 30 minutes. The solvent was removed; the residue was rubbed with ether, filtered, washed with ether on filter and dried *in vacuo* over potassium hydroxide. The yield was 4.54 g (92%) of dipeptide trifluoro-acetate **(8)** with R_{f} 0.37 (D); 0.17 (E).

1.14 g (3 mmol) of dipeptide amide trifluoroacetate **(8),** 1.6 g (3 mmol) of protected dipeptide (**11**) were dissolved in 60 ml of DMF, cooled down to -5°C and 0.51 ml (3 mmol) of DIEA were added upon stirring and cooling and 0.75 ml (3.6 mmol) of DPPA dissolved in 5ml of DMF were slowly dropped on to the mixture. The reaction was carried out for several hours and was left overnight. After the reaction was completed (chromatographic control), the mixture was diluted with ethyl acetate and water, cooled down to 0°C and acidified with 5% KHSO₄ solution to pH 3. The ethyl acetate layer was separated; water phase was extracted with one portion of ethyl acetate additionally. The organic phases were combined, washed with 5% NaHCO₃ solution, water, 5% KHSO₄, water, saturated water solution of NaCl and finally dried over anhydrous Na₂SO₄. The solvent was removed and the product was purified on the silica gel column (C) (the product was loaded in system ch I and the elution was continued in system ch II). The respective fractions were collected, evaporated and dried in vacuo. The yield was 0.73 g (31%) of protected tetrapeptide amide **(12)**. The analytical sample was purified by RP-HPLC. M.p. 169-170°C, [a]_{D}²² -46.8°(c 0.25, DMF), R_{f} 0.51(E, "Merck");0.66(B "Merck").

### Tyr-(N^{δ}-Ac)-D-Orn-Gly-Phe(p-NO₂) -NH₂(1)

0.25 g (31 mmol) of tetrapeptide amide **(12)** was dissolved in 10ml of 50% TFA in DCM and work up continued as for compound **(8).** The product was purified on the Sephadex G-10 column. Elution was performed with 0.02 AcOH solution. The yield was 0.18 g (90%) of tetrapeptide amide **(1)** after lyophilization. M.p. 130-131°C, [a]_{D}²² +80°(c 0.15, 0.2 N, AcOH), R_{f} 0.52(D, "Merck");0.49(B "Merck"); amino acid analysis: Gly 1.00; Tyr 0.86; Orn 0.82; Phe(p-NO₂) 0.91. The analytical HPLC analysis showed one peak with k'=1.6 (column 4.6x110, Silasorb SPH, mobil phase: CH₃CN and 0.2M ammonia acetate buffer (21:79), pH 5, flow rate 1.5 ml/min, λ=220nm.

### Examples 2, 3, 4, 5

### Synthesis of enkephalin analogues (2,3,4,5)

N-protected amino acid derivatives, supports and reagents for peptide synthesis were purchased from Sigma, Fisher Scientific, Bachem; Reanal (Hungary); Saxon Bichemicals GMBH (Germany). Except for 4-methylbenzhydrylaminopolymer which was purchased from Novabiochem, Switzerland. HPLC-grade solvents DCM, DMF (was stored over 4 Å molecular sieve), MeCN and MeOH were used in synthesis and purification. Other reagents were reagent grade. Melting points were determined on a Boetius hot plate (Germany).

Synthesis of the peptides **(2,3,4,5)** were performed by the solid phase methodology utilizing an NPS-4000 semi-automatic synthesizer (Neosystem Laboratories, France) using a standard BOC/Bzl protecting group tactics on MBHA resin.

The α-amino function of the amino acids was protected by the BOC protecting group, and the reactive side-chain functional groups were protected as follows: Ac group for D-Lys; Fmoc group for the D-Dab and D-Orn; and 2-bromobenzyloxycarbonyl (2-Br-Z) for the Tyr amino acid residue. The side chain of Asn was unprotected. All peptides were constructed with an amidated C terminus on the 4-methylbenzhydrylamine resin (0.6 mmol/g resin substitution). A 3-fold excess of Boc-amino acids was used for the coupling reactions. Couplings of the amino acids were performed using N,N'-diisopropylcarbodiimide in dimethylformamide for the coupling of amino acid residues to the growing peptide resin in the presence of 6-Cl-HOBt. After a coupling time of 2 h, the completeness of acylation was monitored at each stage by the standard ninhydrin test. In case of the incomplete coupling, the coupling procedure was repeated before the removal of the Boc protecting groups. Trifluoroacetic acid in DCM (65% (vol/vol) was used for the selective cleavage of the N-protecting group, followed by neutralization with triethylamine in DMF (5% (vol/vol). Acetylation of the side-chain amino group of peptides was achieved by the treatment with a 10% solution of Ac₂O in DCM containing also triethylamine.

Final deprotection as well as the cleavage of the peptides from the resin was performed by the treatment with 1 M solution of trifluoromethanesulfonic acid in TFA in the presence of thioanisole and ethanedithiol.

The synthesized peptides were desalted by gel filtration, purified by RP-HPLC and characterized by mass-spectrometry and amino acid analysis.

Gel filtration was performed on Toyopearl TSK HW-40 column. Solution of 0.1 M NH₄HCO₃ in iPrOH was used for the elution of peptides. Final purification was achieved by RP-HPLC on the System Gold Chromatograph (Beckman, United States) equipped with a Luna C₁₈ column (4.6 x 150 mm) for the analytical chromatography and Vydac C₁₈ column (22 x 250 mm) for the preparative chromatography. The peptides were eluted with a gradient of acetonitrile in 0.1% H₃PO₄ at a flow rate of 1 ml/min (analytical columns) and a gradient of acetonitrile in 0.1% TFA at a flow rate of 10 ml/min, (preparative columns), respectively. UV detection was carried out at 230 nm.

The purity of the amino acid derivatives was controlled by TLC on precoated Merck F 254 plates (Germany) in the following chromatographic systems: 1:3 1% ammonia - sec-butanol, 9:2 chloroform - methanol, and 90:7.3 chloroform - methanol - acetic acid. The purity of synthesized peptides was determined in systems: nBuOH-AcOH-H₂O (4:1:1); CHCl₃-MeOH-AcOH-H₂O (30:20:4:6); (60:18:2:3). Chromatograms were visualized by UV irradiation at 254 nm, by spraying with ninhydrin solution or using chlorine/benzidine reagent. Amino acid analysis was performed on a Microtechna T339M analyzer (Czech Republic) after peptide hydrolysis in a sealed ampoule (24 hrs at 110°C). The mass spectra were recorded on a MX-5303 time-off-flight mass spectrometer at electrospray ionization (FINEPKhF) and reflect mode.

### Biology

### Examples of biological activity

### Cardioprotective action of the enkephalin analogue (1)

Cardioprotective action of the enkephalin analogue (1) according to the invention has been tested on 145 white male rats weighing 200 to 250 g. The sharp myocardial ischemia was created by ligature of the left coronary artery. The compounds studied were injected intraperitoneally to animals at doses of 0.05 mg/kg and 0.5 mg/kg after 30 min. of coronary occlusion. Nitroglycerine and anaprilinum were tested for comparison as the traditional medicines used for myocardial infarction treatment. Rats with sharp myocardial ischemia receiving an injection of physiological solution were used in the control experiments.

The animals were sacrificed 6 and 24 hours after ligature of coronary artery and samples of blood and heart tissue were taken for investigation. The heart was washed from blood, left ventricle was separated from right ventricle and cut into 4-5 uniform transversal segments. All segments were incubated in 0.1 % nitroblue tetrazole solution in phosphate buffer pH 7.4 during 15 min at 37°C. The necrosis area size of the heart muscle was determined by the gravitation method. The rate of ischemic damage was estimated according to the level of creatine phosphokinase (CPK) and its heart fraction (HF-CPK) in blood. Standard kits (Boehringer, Germany) were used to test and calculate the activity of both kinases (CPK+ HF-CPK). The results obtained were statistically processed using Student's criterion.

It was determined that the coronary artery occlusion contributes to appearance of a detectable necrosis area after 6 hours, which gradually spreads during the following 24 hours. Characteristic metabolic changes in blood were observed. Lactate content increases, which shows the degree of the ischemia and circular hypoxia. Enzymatic activity of CPK and HF-CPK increases, which reflects the ischemic cardiomyocite damage (Table 2).

There was no statistically significant difference between the necrosis area size before and after 6 hours after coronary occlusion caused by peritoneal administration of the enkephalin analogue (1) in rats with myocardial infarction compared to rats from the control group.

However, the myocardial necrosis area size of animals injected with the new enkephalin analogue (1) was statistically smaller at the end of the 24 hours of experiment in comparison to animals of the control group (Table 1).

**Table 1. The influence of enkephalin analogues on the myocardial necrosis size of rats with ligature of coronary arteries**

| No. | Groups of animals | Quantity of animals | Observation time | |
|---|---|---|---|---|
| | | | 6 hours | 12 hours |
| | MI +physiological solution | 15 | 43.07 ± 2.91 | 55.80 ± 1.71 |
| 1 | MI+Tyr-D-Orn(Ac)-Gly-Phe(NO₂)-NH₂ 0.05mg/kg | 15 | 40.70 ± 1.52 P >0.05 | 47.51 ± 1.63 P > 0.05 |
| 1 | MI+Tyr-D-Orn(Ac)-Gly-Phe(NO₂)-NH₂ 0.5mg/kg | 15 | 40.05 ± 1.93 P > 0.05 | 46.64 ± 2.20 P > 0.05 |
| | MI+nitroglycerine | 15 | | 54.04 ± 1.76 P > 0.05 |
| | MI+anaprilinum | 15 | | 53.80 ± 1.90 P > 0.05 |

The lactate content in the blood is the main reliable.index of myocardial infarction proceeding. It was determined that a dose of 0.05 mg/kg of the new enkephalin analogue (1) decreases development of hyperlactatemia and hyperactivity of the enzymes CPK and HF-CPK in blood. The level of glycogen (the main energy store) of heart and liver increases (Table 2). This demonstrates the beneficial influence of compound (1) on the pathological process. The reduced lethality of experimental animals is just a proof for that (see Table 3).

It means also that the new enkephalin analogue (1) increases the organism stability to oxygen -dependent pathological conditions (for example, shock, ischemia, bleeding etc.)

**Table 2. Metabolic results of blood and tissue testing of animals with sharp myocardial ischemia, obtaining physiological solution, enkephalin analogue (1) or nitroglycerine**

| Data | Groups of animals | | | | | |
|---|---|---|---|---|---|---|
| | Control | MI +physiological solution | | MI+compound (1) | | MI+nitroglycerine |
| | | 6 hours n=12 | 24 hours n=11 | 6 hours n=11 | 24 hours n=10 | 24 hours n=12 |
| Lactate (mmol/l) | 1.44 ± 0.07 | 4.03 ± 1.26 P<0.05 | 2.01 ± 0.05 P<0.05 | 1.61 ± 0.45 P > 0, 05 | 1.70 ± 0.09 P<0.05 | 1.80± 0.09 P<0.05 |
| CPK units/I | 68.3 ± 5.8 | 134.6 ± 7.6 P<0.05 | 108.8 ± 6.2 P<0.05 | 93.5 ± 5.3 P<0.05 | 86.6 ± 4.9 P<0.05 | 101.1 ± 6.1 P<0.05 |
| HF-CPK units/I | 11.9 ± 1.1 | 18.1 ± 0.6 P<0.05 | 26.6 ± 2.1 P<0.05 | 15.3 ± 0.7 P<0.05 | 12.3 ± 0.7 P<0.05 | 19.8 ± 1.1 P<0.05 |
| Glycogen of heart, g/kg | 1.35 ± 0.13 | 0.41 ± 0.06 P<0.05 | 0.46 ± 0.07 P<0.05 | 1.00 ± 0.12 P > 0, 05 | 1.11 ± 0.17 P > 0, 05 | |
| Glycogen of liver, g/kg | 6.94 ± 0.44 | 4.85 ± 0.62 P<0.05 | | 6.98 ± 1.02 P > 0, 05 | | |

**Table 3. The lethality of rats with sharp myocardial ischemia obtaining physiological solutions and enkephalin analogue (1)**

| Groups of animals | N | Lethality % |
|---|---|---|
| MI+ physiological solution | 30 | 53 |
| MI+ compound (1), 0.05mg/kg | 30 | 33 |
| MI+ compound (1), 0.5mg/kg | 30 | 27 |

The results show that the new enkephalin analogue **(1)** according to the invention has favourable influence on the process of experimental myocardial infarction.

Morphological investigation and the study of the enzyme level in blood demonstrate the cardioprotective action and the ability to limit the spreading of necrosis area size of the heart muscle.

Nitroglycerine and anaprilinum in highest therapeutic doses were used for comparison with the medicines traditionally used for myocardial infarction treatment. It was shown that the decrement rate of hyperfermentemia and the decrement of the necrosis area size of the myocardium (Table 1 and 2) of animals injected by nitroglycerine and anaprilinum were much smaller than that when the new enkephalin analogue (1) was injected. It was concluded that the new enkephalin analogue (1) more effectively decreases the extent of ischemic damage of the heart muscle than the known cardioprotective compunds, nitroglycerine and anaprilinum.

The new enkephalin analogue (1) possesses anti-ischemic action and increases the organism's stability to hypoxia. It limits the spreading of the necrosis area size of the heart muscle of animals with experimental myocardial infarction and decreases the lethality in experimental animals.

### Cardioprotective activity assay in vivo of the enkephalin analogue (4).

The rats for this study were used from the Lab Animals Breeding Centre and were maintained in accordance with the policies and guidelines of the position of the Committee on Care and Use of Laboratory Animals of the Institute of Bioorganic Chemistry (Branch), Russian Academy of Science (IBC RAS). The Test Facility was followed requirements specified in the Standard Operating Procedures (SOPs) of the Laboratory.

### Cardiac ischemia rat model

### Animal preparation.

### Surgery and Left Coronary Artery Occlusion. (LCAO)

Male Sprague-Dawley (CD) rats weighing 280-380 g were used throughout this study. The rats were anesthetized by i.p. administration of urethane (1-1.5 g/kg). A tracheotomy was performed, and the trachea of the animal was intubated with a cannula connected to a rodent ventilator (model UGO BASILE 7025). Body temperature was maintained at 37°C by use of a heating pad and monitored using rectally placed temperature probes connected to a digital thermometer. Body temperature is monitored and maintained at 37±1°C)

The femoral artery was isolated and cannulated to measure blood pressure (BP) by a BP transducer (model PN:1891-E45, DTX™, Singapore) connected with computer. Mean arterial pressure and heart rate was calculated by special software. The right femoral vein was cannulated to allow administration of peptides or saline and methylene blue dye.

A left thoracotomy was performed to expose the heart at the fifth intercostal space. The pericardium was removed and a suture was placed under the left coronary artery in the groove between the pulmonary outflow track and left ventricle. With the tubing placed on the myocardial surface, the ligature could be tightened around the left coronary artery and clamped to provide a readily reversible occlusion of blood flow. Hemodynamics and body temperature were monitored during the procedure.

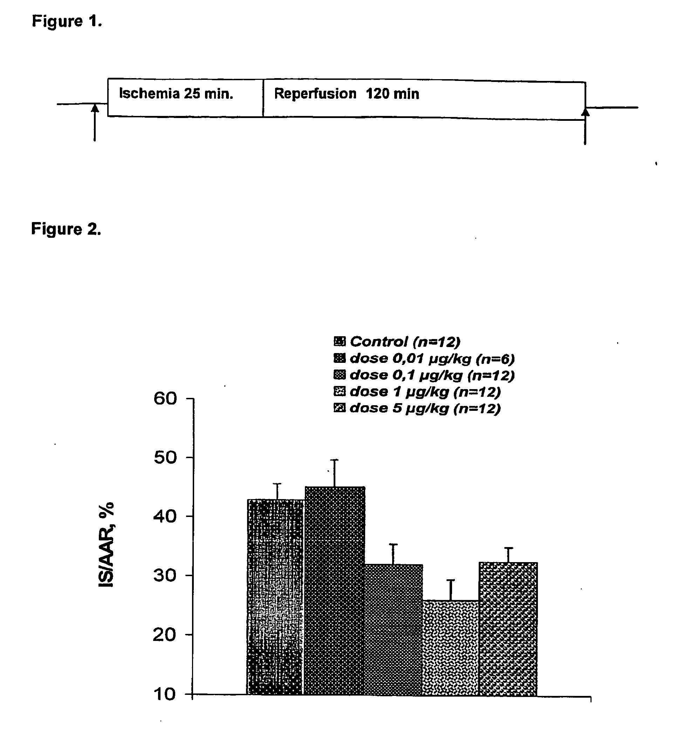

The protocol of experiments conducted in urethane-anesthetized open-chest rats subjected to regional myocardial ischemia-reperfusion is illustrated in Figure 1 (see appendix).

All animals were subjected to 25 minutes of occlusion and 2 hours of reperfusion. Several doses (0.1 µg/kg-1µg/kg) of the peptide (4) were given 5 minutes before occlusion. The coronary artery in all rats was occluded by pulling up on the suture and clamping it with plastic tube. At 25 minutes, the clump was released and coronary artery reperfusion was allowed. The surface of the left ventricle pinks up. After 120 min of reperfusion, the ligature was again tightened to occlude the left coronary artery, and 2 % methylene blue dye (0.4 ml of 10% w/v in saline) was injected. Once the dye had stained the heart, except in the area at risk, clearly demarcating the total area at risk (AAR), the heart was then quickly removed from the animal and washed out in a water, and the atria, valvular material and right ventricular wall were removed leaving just the left ventricle. The left ventricle was then sectioned transversely into 2-mm slices. The area defined as normal (dyed blue) was separated from the AAR (not dyed blue), and the slices are stained by incubation in 1% 2,3,5-triphenyltetrazolium chloride in pH 7.4 buffer for 15 min at 37°C. Infarcted areas appeared whitish after 2,3,5-triphenyltetrazolium chloride staining, whereas non-infarcted, or viable, areas appeared reddish in colour. Then the slices were immersed in 10% formalin for 5 minutes for fixing.

### Measurement of the area at risk and infarct size

The slices were photographed. The areas of infarct (2,3,5-triphenyltetrazolium chloride negative) and risk zone (2,3,5-triphenyltetrazolium chloride stained) for each slice are traced and digitized using a computerized planimetry technique. The areas of each region were estimated using computerized planimetry (program "ImageTool")

The infarct area was divided by the area at risk to determine the ratio of the infarct area/area at risk. (IS/AAR). Data were presented as infarct size (IS) as a percentage of the AAR (i.e., %IS/AAR) for statistical analysis of cardioprotective effects (decreased %IS/AAR). The statistical comparison for *in vivo* infarct reduction was made with vehicle controls.

### Experimental protocol

A dose-response curve for the cardioprotective effects of peptide **(4)**, compared with vehicle (*n* = 12), was produced with i.v. peptide **(4)** doses of 0.01 (*n* = 6), 0.1 (*n* = 12), 1.0 (*n* = 12) µg/kg, and 0.005 (*n* = 12) mg/kg administered 5 min before LCAO in a 1 ml/kg dosing volume as a bolus.

The influence of peptide **(4)** on reperfusion injury after ischemia was assessed through the administration of 1.0 µg/kg peptide **(4)** (1 ml/kg bolus) immediately after (0 min; *n* = 6) removal of the LCAO.

### Results

### Modelling of Cardioprotective Effects: Dose-Response Curve.

Control animals demonstrated a mean infarct size of 42.9 ± 2.7. Peptide **(4)** significantly reduced infarct size expressed as a % of Area at Risk (IS/AAR) (P < 0.05; Table 4) when administrated 5 min before ischemia and was smaller than 30% at a doses between 0.2 µg/kg and 3.2 µg/kg. The effect of peptide **(4)** was maximal at a dose of 1.0 µg/kg (26,0 ± 3,5%). Intravenous administration of peptide **(4)** at a low dose of 0.01 µg/kg had no effect on infarct size (45.1 ± 4.6%) (Table 4 and Figure 2 in appendix).

### Effects of Peptide (4) on Reperfusion Injury.

When administered immediately after removal of the LCAO (i.e., at the start of the 120-min reperfusion period), peptide **(4)** significantly reduced the level of ischemic damage compared with controls (32.5±2,4 %, mean ± S.E.M.; Figure, 3 see appendix).

We conclude that peptide **(4)** limits ischemic injury *in vivo*, when administered i.v. to male Sprague-Dawley rats 5 min before a 25 min occlusion and 120 min reperfusion period. Peptide **(4)** (dose 1.0 µg/kg) reduced the infarct size by approximately 39% compared with saline-treated controls (Table **4).** The beneficial effects on infarct size showed the potential for clinical use before surgically induced ischemia and in case of emergency.

**Table 4. Infarct sizes data in rat hearts treated with peptide (4) . Infarct sizes as a % of Area at Risk (IS/AAR)**

| No. | Groups of animals | Quantity of animals, n | IS/AAR,% | Relative Reduction of Infarct size, % (Control=0) |
|---|---|---|---|---|
| 1 | MI + physiological solution P< 0.05 | 12 | 42.9 ± 2.7 | 0 |
| 2 | MI +Peptide **(4)** Dose 0,01 µg/kg | 6 | 45.1 ± 4.6 | |
| 3. | MI +Peptide **(4)** Dose 0,1 µg/kg P =0.022 | 12 | 32,0 ± 3,5 | 25.4 |
| 4 | MI +Peptide **(4)** Dose 1 µg/kg P =0.001 | 12 | 26,0 ± 3,5 | 39.4 |
| 5 | MI +Peptide **(4)** Dose 5 µg/kg P =0.010 | 12 | 32,5 ± 2,5 | 24.2 |

### Antihypoxic action

Antihypoxic action of the new enkephalin analogue (1) was determined by the measurement of lifetime duration in mice under the normobaric hypoxic hypoxia conditions.

The experiments were conducted on BALB-bread mice weighing 20 to 22 g. The normobaric hypoxic hypoxia conditions were modelled by placing the animals in hermetic camera, containing Ca(OH)2 to absorb the excess of carbon dioxide. The substances studied were injected intraperitoneally 1 min. before the placing of animals in the hermetic camera. The peptides were used at doses 1.0 mg/kg. Such a dose was found to be optimal to study antihypoxic properties of peptides. Sodium oxybutyrate at highest therapeutic dose was used as comparative compound. The lifetime was estimated as the time when the animals stopped making breathing movements. The results are shown in the Table 5.

**Table 5. The influence of enkephalin analogues and sodium oxybutyrate on live duration of mouse at hypoxic hypoxigenation**

| No | Compounds Injected | Dose mg/kg | Quantity of animals | Duration of life min | P |
|---|---|---|---|---|---|
| | Control (physiological solution) | | 20 | 10.7 ± 0.4 | |
| 1 | Tyr-D-Orn(Ac)-Gly-Phe(NO₂)-NH₂ | 1.0 | 22 | 16.9 ± 1.1 | 0.01 |
| | Control | | 20 | 12.5 ± 0.3 | |
| | Sodium oxybutyrate | 1000.0 | 20 | 15.5 ± 0.4 | 0.01 |

The lifetime of the animals injected with the new enkephalin analogue (1) was 57% longer than in the control experiments. Injection of sodium oxybutyrate increased the lifetime duration of animals by 24% in comparison with the control experiments.

### Analgesic activity assay in vivo of the enkephalin analogues (1)

### "Tail pinch" method

Analgesic activity of enkephalin analogues was determined by a "tail pinch" method [Ueda H.; Amano H.; Shiomi H.; Takagi H. (1979). Eur. J. Pharmacol., 56, 265-268]. Analgesic effect was studied in comparison with morphine, [Leu⁵]- and [Met⁵]-enkephalins at intracisternal and intravenous administration to outbreed male mice weighing 18 to 22g. Ten mice were used in each experiment.

The studied substances were dissolved in sterile physiological solution and injected in J-shaped needles into the brain of conscious mice at a dose of 10 µl. Control animals received 10 µl of sterile physiological solution. Intravenous administration was into the tail vein. Analgesic activity was tested by pinching the mouse tail with an artery clip with 200 g pressure. The mice who bit the clip within one second after application of pinching were selected for assays. Analgesic activity was defined as latency on the biting response greater than 6 sec. The analgesic tests were performed 5, 15, 30, 60 and 90 min after administration and then every 30 min until the analgesic reaction was stopped. The analgesic activity of the peptides was expressed as a percentage of mice not showing the biting response. The results are summarised in Tables 6 and 7. Analgesic activity is shown as relative to morphine (morphine = 1)

**Table 6. Analgesic Activity of Enkephalin Analogues at intracisternal administration (method "tail pinch")**

| | | | Duration of | Relative anal- |
|---|---|---|---|---|
| No | Compound | ED₅₀ nmol/animal | analgesic effect | gesic activity |
| | | (dose interval) | at ED₆₀₋₈₀, min | morphine = 1 |
| | | | | |
| 1 | Tyr-D-Om(Ac)-Gly-Phe(NO₂)-NH₂ | 0.02 (0.01-0.04) | 120 | 60 |
| a) | [Leu⁵]-enkephalin | 174 (102-281) | 15 | 0.007 |
| b) | [Met⁵]-enkephalin | 154 (90-261) | 15 | 0.008 |
| c) | Morphine | 1.2 (0.6-2.2) | 60 | 1 |

**Table 7. Analgesic Activity of Enkephalin Analogues at intravenous administration (method "tail pinch")**

| | | | Duration of | Relative anal- |
|---|---|---|---|---|
| No | Compound | ED₅₀ µmol/kg | analgesic effect | gesic activity |
| | | (dose interval) | At ED₆₀₋₈₀, min | morphine = 1 |
| | | | | |
| 1 | Tyr-D-Orn(Ac)-Gly-Phe(NO₂)-NH₂ | 8.0 (0-25) | 60 | 2,2 |
| | | | | |
| c) | Morphine | 18 (15-22) | 60 | 1 |

As shown in Table 6 the new enkephalin analogue **(1)** possess a pronounced analgesic effect; it is much more potent than native enkephalins (compounds a),b)) and 60 times more potent than opioid standard - morphine after intracisternal administration.

Unlike native enkephalins the new enkephalin analogue **(1)** was active after intravenous administration. It was 2 times more active than morphine after intravenous administration (see Table 7).

### Analgesic activity assay in vivo of the enkephalin analogues (1,2,3,4,5). "Hot plate" method

This study was conducted in compliance with current U.S. FDA Good Laboratory Practice (GLP) Regulations for Non-clinical Laboratory Studies (21 CFR Part 58). The Test Facility was followed requirements specified in the Standard Operating Procedures (SOPs) of the Laboratory.

Analgesic activities of the peptides **(1-5)** were assayed in CD-1 male mice by means of the hot-plate test (G.Woolfe and A.D.Macdonald, J.Pharmacol.Exp.Ther., 80, 300 (1944)) after intravenous administration. Morphine has been tested as a compound for comparison.

Male Swiss albino mice (CD1), weighing 20-22 g were used throughout the study. The animals were housed in solid bottom polycarbonate cages with access to standard laboratory diet (Laboratory Rodent Diet PK-120) and tap water ad libitum, under controlled environmental conditions (temperature: 18-26°C, 8 a.m. to 8 p.m. light-dark cycle). Mice were tested only once.

The Hot-plate-meter represents the thermostatic controllable, electro-heated up surface limited by transparent walls (L: 25 cm, W: 25 cm, H: 35 cm). The forward panel of the device is equipped by buttons for the control of time and a temperature mode (Columbus Instruments).

All peptides and morphine for intravenous (i.v.) administration were dissolved in saline. The i.v. administration was performed via tail vein as a bolus injection. The doses for all animals were calculated on the basis of the last value of the individual body weight.

The animals accurately were placed on the preheated hot plate (55±0.5°C) after i.v. injection of saline or peptides and the latencies to first paw licking and first jumping were measured. The other behavioral reactions were ignored. The animals were removed from a hot surface after the first jumping. A cut-off time of 180 s was used to avoid tissue damage.

To evaluate the hot-plate test responses, the control latencies (t₀) and test latencies (t₁) were determined after injection of saline or peptides, respectively. The percentage of the maximal possible effect (%MPE) was calculated as % MPE= (t₁-t₀) / (t₂-t₀) x100, where the cut-off time (t₂) was 180 s.

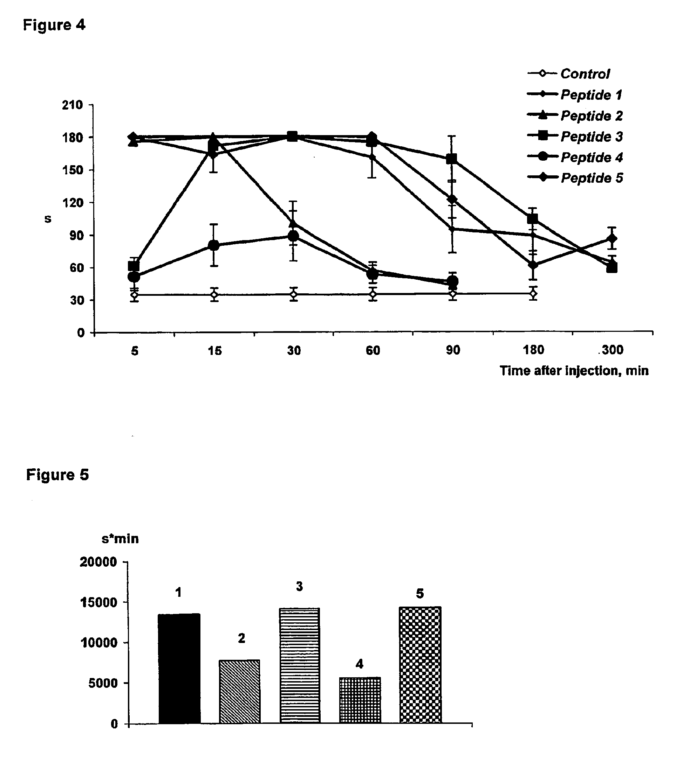

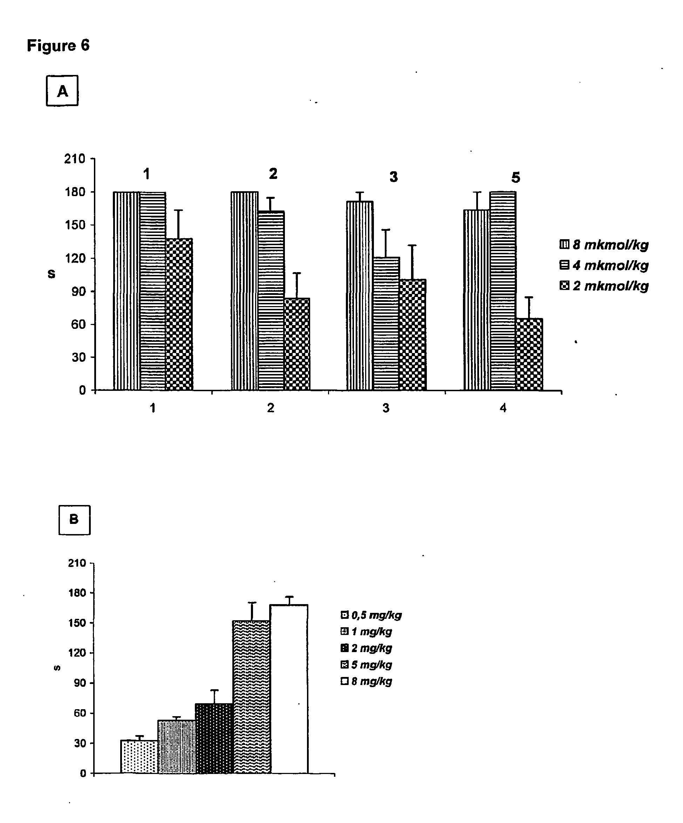

The latency for the first jumping is considered as the most indicative in the hot-plate test. The analgesic activity of peptides **(1-5)** (dose 8 µmol/kg) expressed as jump latency time are presented in Figure 4 (see appendix). The area under the time-response curve was calculated by methods described elsewhere (Cicero and Meyer, 1973), to assess the magnitude of antinociception (Figure 5, see appendix).
The results in comparison with morphine are shown in Figure 6 (see appendix).

The median analgesic dose (ED₅₀) also has been calculated for this parameter. The results of the analgesic activity of peptides (1,2,3,5) in the mouse hot-plate test after intravenous administration at the time point of 15 min are shown in Table 8.

As shown in Figure 4 (see appendix) the enkephalin analogues **(1-5)** possess a pronounced analgesic effect lasting up to 90-300 min after i.v. administration to mice ("hot plate" method). Peptide **(1)** and **(5)** show similar profiles whereas peptide **(3)** showed a 15 min delayed effect and after that similar profile as peptide **(1)** and **(5).** Peptide **(4)** demonstrated marked analgesic effect with the maximum value at the 30 min. However, analgesic effect of the peptide **(4)** was weaker than the effect of the other four peptides tested.

**Table 8. The analgesic activity of peptides (1,2,3,5) in the mouse hot-plate test after intravenous administration at the time point of 15 min**

| **ED₅₀, mg/kg** | | | | |
|---|---|---|---|---|
| **Morphine** | **Peptide 1** | **Peptide 2** | **Peptide 3** | **Peptide 5** |
| **2,67** | **1,27** | **1,7** | **2,04** | **1,99** |

In the hot-plate test, peptides **(1-5),** the new synthetic enkephalin analogues possessed the pronounced analgesic activity. Peptides **(3)** and **(5)** revealed an analgesic potency practically similar to that of morphine.
Peptides **(1)** (ED₅₀ **=1,27 mg/kg**) and **(2)** (ED₅₀ **=1,7 mg/kg**) turn out to be the most active. The analgesic activity of peptides (1) and (2) exceeded that of the morphine by 2 and 1.5 times, respectively (Table 8).

### Analgesic activity assays in vitro

### GPI and MVD bioassays

The effect of peptides on peripheral opiate receptors was determined by the capacity to suppress electric stimulation-induced segment contraction of the longitudinal muscle of the guinea pig ileum with mesenteral nervous plexus and of the vas deferens of mice [Paton W.D.M.; Visi E.S. (1969). Brit. J. Pharmacol., 35, 10-28]. The longitudinal muscle of ileum from guinea pig of any sex (weight 350-500 g) was carefully separated from underlying circular muscle and placed into a small bath with Krebs solution at 36°C.

The solution was constantly aerated. A constant load of 0.2 g was applied to the tissue. The tissue was stimulated by means of the ring platinum electrodes by single impulses of 1 µsec duration with 0.1 Hz frequency. Isometric contractions were registered on a TB-611T recorder connected to a Nihon Kohden polygraph. Vas deferens of mice (weight 27 to 30 g) were placed into a bath with modified Krebs solution (not containing magnesium sulphate) at 31°C. The substances under study were dissolved in distilled water. The inhibiting activity of the compounds was determined by accumulating doses, adding increasing concentrations of the compounds without washing. The activity of the preparations was expressed as IC₅₀ in nmol/l. The results obtained in 8-10 tests were statistically processed using Student's criterion. Table 9 demonstrates IC₅₀ with reliable intervals at P 0.05.

**Table 9. Opioid activity of Enkephalin Analogue in the Guinea Pig Ileum (GPI) and Mouse Vas Deferens (MVD)**

| No | Compound | GPI | | MVD | | |
|---|---|---|---|---|---|---|
| | | IC₅₀ nM | Relative potency, % | IC₅₀ Nm | Relative potency, % | GPIᵣₑₗ, / MVDᵣₑₗ |
| 1 | Tyr-D-Orn(Ac)-Gly-Phe(NO₂)-NH₂ | 12.0 ± 2.0 | 2375 | 38.5 ± 20.05 | 52 | 46 |
| a) | [Leu⁵]-enkephalin | 285 ± 78 | 100 | 20 ± 1 | 100 | 1 |
| c) | Morphine | 78 ± 19 | 365 | 579 ± 224 | 3 | 107 |

In the GPI assay (predominantly µ-type of opioid receptor) the enkephalin analogue (1) demonstrated high level of potency. Its µ-affinity exceeded the affinity of [Leu⁵]-enkephalin by 23 times. The enkephalin analogue (1) was also more active than morphine by one order.

### Conclusions

Thus we have shown the pronounced cardioprotective activity of the new peptides (the reduction of the infarct size by 24-39 % ), better protection against ischemic damage to heart muscle than that provided other known cardioprotective medicines such as nitroglycerine and the β-adrenergic blocking agent anaprilinum, more pronounced antihypoxic action than that of known pharmacological medicines, such as sodium oxybutyrate, and a more pronounced and long-lasting analgesic activity than that of morphine at intravenous administration.

## Claims

1. A compound comprising the tetrapeptide structure:
**A-B-C-D**
wherein
each hydrogen of the N-terminus NH₂ of amino acid residue **A** may independently optionally be replaced by C₁₋₅ alkyl or C₁₋₅ acyl,
**A** - is an aromatic amino acid residue wherein the aromatic ring may optionally be substituted with one or more nitro group,
**B** - is a diamino acid residue wherein the side chain amino group is substituted with C₁₋₅ acyl, C₁₋₅ alkyl, -**B'**-C₁₋₅ acyl or **-B'**-C₁₋₅ alkyl, wherein **B'** is an amino acid residue which may optionally be substituted with one or more nitro group,
**C** - is Gly wherein the peptide N-H may optionally be changed to N-methyl,
**D** - is Phe which is substituted on the aromatic ring with at least one nitro group and optionally further substituted on the aromatic ring with one or more group independently selected from nitro, fluoro, chloro, bromo, iodo, CF₃ or CN, wherein the peptide N-H may optionally be changed to N-methyl,
and the C-terminus C=O of amino acid residue **D** is substituted with NH₂, NHC₁₋₅ alkyl, NH-NH₂, NH-NHC₁₋₅ alkyl, or O-C₁₋₅ alkyl; or the C-terminus is COOH or CH₂OH,
or a pharmaceutically acceptable salt thereof.

2. A compound as claimed in claim 1, wherein the diamino acid residue in position **B** is in a D- or L-configuration.

3. A compound as claimed in claim 1 wherein the N-terminus NH₂ is unsubstituted.

4. A compound as claimed in any preceding claim wherein
**A -** is selected from Tyr, Thyronine, Phe or Trp, wherein the aromatic ring may optionally be substituted with one or more nitro group, and preferably **A** is unsubstituted Tyr.

5. A compound as claimed in any preceding claim wherein
**B** - is Dap, Dab, Orn, Lys, 4,5-dehydro-lysine or 2,6-diamino-4-hexynoic acid, wherein the side chain amino group is substituted with C₁₋₅ acyl, C₁₋₅ alkyl, **-B'**-C₁₋₅ acyl or **-B'**-C₁₋₅ alkyl.

6. A compound as claimed in any preceding claim wherein
**B** - is Dab, Orn or Lys, wherein the side chain amino group is substituted with C₁₋₅ acyl, C₁₋₅ alkyl, **-B'**-C₁₋₅ acyl or **-B'**-C₁₋₅ alkyl, and preferably B is Orn substituted with C₁₋₅ acyl.

7. A compound as claimed in any preceding claim wherein the side chain amino group of **B** is substituted with C₁₋₅ acyl or **-B'**-C₁₋₅ acyl, preferably C₁₋₅ acyl, more preferably acetyl.

8. A compound as claimed in any preceding claim wherein
**B'** - is Gly, Ala, Pro, Leu, Asn or Met, preferably Gly or Asn.

9. A compound as claimed in any preceding claim wherein
**C** - is unsubstituted Gly.

10. A compound as claimed in any preceding claim wherein
**D** - is Phe substituted at the para-position with nitro and optionally further substituted on the aromatic ring with one or more group independently selected from nitro, fluoro, chloro, bromo, iodo, CF₃ or CN, wherein the peptide N-H may optionally be changed to N-methyl, and preferably the only substituent on the aromatic ring is para-nitro.

11. A compound as claimed in any preceding claim wherein
the C-terminus C=O of amino acid residue **D** is substituted with NH₂.

12. A compound as claimed in claim 1, comprising the following structure:
Tyr-D-Orn(Ac)-Gly-Phe(p-NO₂)-NH₂,

13. A compound as claimed in claim 1, selected from the compounds comprising the following structures:
Tyr-D-Lys(Ac)-Gly-Phe(p-NO₂)-NH₂ (2)
Tyr-D-Orn(Ac-Asn)-Gly-Phe(p-NO₂)-NH₂ (3)
Tyr-D-Dab(Ac)-Gly-Phe(p-NO₂)-NH₂ (4)
Tyr-D-Orn(Ac-Gly)-Gly-Phe(p-NO₂)-NH₂ (5)

14. A pharmaceutical composition comprising a compound as claimed in any preceding claim and a pharmacologically acceptable diluent, carrier or excipient.

15. A compound as claimed in any of claims 1 to 13 or a pharmaceutical composition as claimed in claim 14 for use as a medicament.

16. Use of a compound as claimed in any of claims 1 to 13 for the manufacture of a medicament for tissue protective treatment, preferably cardioprotective treatment.

17. Use of a compound as claimed in any of claims 1 to 13 for the manufacture of a medicament for antihypoxic treatment.

18. Use of a compound as claimed in any of claims 1 to 13 for the manufacture of a medicament for analgesic treatment.

19. Use of a compound as claimed in any of claims 1 to 13 for the manufacture of a medicament for anti-ischemic treatment.

20. Use of a compound as claimed in any of claims 1 to 13 for the manufacture of a medicament for tissue protective treatment, preferably cardioprotective treatment, in combination with either or both of antihypoxic or analgesic treatment.

21. A compound as claimed in any of claims 1 to 13 for use in tissue protective treatment, preferably cardioprotective treatment.

22. A compound as claimed in any of claims 1 to 13 for use in antihypoxic treatment.

23. A compound as claimed in any of claims 1 to 13 for use in analgesic treatment.

24. A compound as claimed in any of claims 1 to 13 for use in anti-ischemic treatment.

25. A compound as claimed in any of claims 1 to 13 for use in tissue protective treatment, preferably cardioprotective treatment, in combination with either or both of antihypoxic or analgesic treatment.

## Patentansprüche

1. Verbindung, umfassend die Tetrapeptid-Struktur:
**A-B-C-D**
worin:
jeder Wasserstoff des N-Terminus NH₂ von Aminosäurerest **A** unabhängig gegebenenfalls durch C₁₋₅-Alkyl oder C₁₋₅-Acyl ersetzt sein kann,
**A** - ein aromatischer Aminosäurerest ist, in dem der aromatische Ring gegebenenfalls mit einer oder mehreren Nitrogruppen substituiert sein kann,
**B** - ein Diaminosäurerest ist, in dem die Seitenketten-Aminogruppe mit C₁₋₅-Acyl, C₁₋₅-Alkyl, **-B'** -C₁₋₅-Acyl oder **-B'**-C₁₋₅-Alkyl substituiert ist, wobei **B'** ein Aminosäurerest ist, der gegebenenfalls mit einer oder mehreren Nitrogruppen substituiert sein kann,
**C** - Gly ist, wobei das Peptid-N-H gegebenenfalls in N-Methyl geändert sein kann,
**D** - Phe ist, das am aromatischen Ring mit wenigstens einer Nitrogruppe substituiert ist und gegebenenfalls außerdem am aromatischen Ring mit einer oder mehreren Gruppen, unabhängig ausgewählt aus Nitro, Fluor, Chlor, Brom, Iod, CF₃ und CN, substituiert ist, wobei das Peptid-N-H gegebenenfalls in N-Methyl geändert sein kann,
und wobei der C-Terminus C=O von Aminosäurerest **D** mit NH₂, NHC₁₋₅-Alkyl, NH-NH₂, NH-NHC₁₋₅-Alkyl oder O-C₁₋₅-Alkyl substituiert ist oder der Terminus COOH oder CH₂OH ist,
oder ein pharmazeutisch verträgliches Salz davon.

2. Verbindung, wie sie in Anspruch 1 beansprucht ist, wobei der Diaminosäurerest in Position **B** in einer D- oder L-Konfiguration ist.

3. Verbindung, wie sie in Anspruch 1 beansprucht ist, wobei der N-Terminus NH₂ unsubstituiert ist.

4. Verbindung, wie sie in einem vorangehenden Anspruch beansprucht ist, wobei
**A** - aus Tyr, Thyronin, Phe und Trp ausgewählt ist, wobei der aromatische Ring gegebenenfalls mit einer oder mehreren Nitrogruppen substituiert sein kann und **A** vorzugsweise unsubstituiertes Tyr ist.

5. Verbindung, wie sie in einem vorangehenden Anspruch beansprucht ist, wobei
**B** - Dap, Dab, Orn, Lys, 4,5-Dehydrolysin oder 2,6-Diamino-4-hexinsäure ist, wobei die Seitenketten-Aminogruppe mit C₁₋₅-Acyl, C₁₋₅-Alkyl, **-B'**-C₁₋₅-Acyl oder **-B'**-C₁₋₅-Alkyl substituiert ist.

6. Verbindung, wie sie in einem vorangehenden Anspruch beansprucht ist, wobei
**B** - Dab, Orn oder Lys ist, wobei die Seitenketten-Aminogruppe mit C₁₋₅-Acyl, C₁₋₅-Alkyl, **-B'**-C₁₋₅-Acyl oder **-B'**-C₁₋₅-Alkyl substituiert ist und B vorzugsweise Orn, substituiert mit C₁₋₅-Acyl, ist.

7. Verbindung, wie sie in einem vorangehenden Anspruch beansprucht ist, wobei die Seitenketten-Aminogruppe von **B** mit C₁₋₅-Acyl oder **-B'**-C₁₋₅-Acyl, vorzugsweise C₁₋₅-Acyl, bevorzugter Acetyl, substituiert ist.

8. Verbindung, wie sie in einem vorangehenden Anspruch beansprucht ist, wobei
**B'** - Gly, Ala, Pro, Leu, Asn oder Met, vorzugsweise Gly oder Asn, ist.

9. Verbindung, wie sie in einem vorangehenden Anspruch beansprucht ist, wobei
C - unsubstituiertes Gly ist.

10. Verbindung, wie sie in einem vorangehenden Anspruch beansprucht ist, wobei
**D** - Phe ist, das an der para-Position mit Nitro substituiert ist und gegebenenfalls außerdem am aromatischen Ring mit einer oder mehreren Gruppen, unabhängig ausgewählt aus Nitro, Fluor, Chlor, Brom, Iod, CF₃ und CN, substituiert ist, wobei das Peptid-N-H gegebenenfalls in N-Methyl geändert sein kann und vorzugsweise der einzige Substituent am aromatischen Ring para-Nitro ist.

11. Verbindung, wie sie in einem vorangehenden Anspruch beansprucht ist, wobei der C-Terminus C=O von Aminosäurerest **D** mit NH₂ substituiert ist.

12. Verbindung, wie sie in Anspruch 1 beansprucht ist, umfassend die folgende Struktur:
Tyr-D-Orn(Ac)-Gly-Phe(p-NO₂)-NH₂

13. Verbindung, wie sie in Anspruch 1 beansprucht ist, ausgewählt aus den Verbindungen, umfassend die folgenden Strukturen:
Tyr-D-Lys(Ac)-Gly-Phe(p-NO₂)-NH₂ (2)
Tyr-D-Orn(Ac-Asn)-Gly-Phe(p-NO₂)-NH₂ (3)
Tyr-D-Dab(Ac)-Gly-Phe(p-NO₂)-NH₂ (4)
Tyr-D-Orn(Ac-Gly)-Gly-Phe(p-NO₂)-NH₂ (5)

14. Pharmazeutische Zusammensetzung, umfassend eine Verbindung, wie sie in einem vorangehenden Anspruch beansprucht ist, und ein pharmakologisch verträgliches Verdünnungsmittel, einen pharmakologisch verträglichen Träger oder ein pharmakologisch verträgliches Exzipiens.

15. Verbindung, wie sie in einem der Ansprüche 1 bis 13 beansprucht ist, oder eine pharmazeutische Zusammensetzung, wie sie in Anspruch 14 beansprucht ist, zur Verwendung als Medikament.

16. Verwendung einer Verbindung, wie sie in einem der Ansprüche 1 bis 13 beansprucht ist, für die Herstellung eines Medikaments zur Gewebe schützenden Behandlung, vorzugsweise kardioprotektiven Behandlung.

17. Verwendung einer Verbindung, wie sie in einem der Ansprüche 1 bis 13 beansprucht ist, für die Herstellung eines Medikaments zur Antihypoxie-Behandlung.

18. Verwendung einer Verbindung, wie sie in einem der Ansprüche 1 bis 13 beansprucht ist, für die Herstellung eines Medikaments zur Schmerzbehandlung.

19. Verwendung einer Verbindung, wie sie in einem der Ansprüche 1 bis 13 beansprucht ist, für die Herstellung eines Medikaments zur antiischämischen Behandlung.

20. Verwendung einer Verbindung, wie sie in einem der Ansprüche 1 bis 13 beansprucht ist, für die Herstellung eines Medikaments zur Gewebe schützenden Behandlung, vorzugsweise kardioprotektiven Behandlung, in Kombination mit einer oder beiden, Antihypoxie-Behandlung oder Schmerzbehandlung.

21. Verbindung, wie sie in einem der Ansprüche 1 bis 13 beansprucht ist, zur Verwendung in einer Gewebe schützenden Behandlung, vorzugsweise kardioprotektiven Behandlung.

22. Verbindung, wie sie in einem der Ansprüche 1 bis 13 beansprucht ist, zur Verwendung in einer Antihypoxie-Behandlung.

23. Verbindung, wie sie in einem der Ansprüche 1 bis 13 beansprucht ist, zur Verwendung in der Schmerzbehandlung.

24. Verbindung, wie sie in einem der Ansprüche 1 bis 13 beansprucht ist, zur Verwendung in einer antiischämischen Behandlung.

25. Verbindung, wie sie in einem der Ansprüche 1 bis 13 beansprucht ist, zur Verwendung in einer Gewebe schützenden Behandlung, vorzugsweise kardioprotektiven Behandlung, in Kombination mit einem oder beiden, Antihypoxie-Behandlung und Schmerzbehandlung.

## Revendications

1. Composé comprenant la structure tétrapeptidique :
A-B-C-D
dans laquelle
chaque hydrogène du NH₂ N-terminal du résidu d'acide aminé A peut indépendamment être éventuellement remplacé par un alkyle en C₁₋₅ ou un acyle en C₁₋₅,
A - est un résidu d'acide aminé aromatique dans lequel le cycle aromatique peut éventuellement être substitué avec un ou plusieurs groupes nitro,
B - est un résidu d'acide diaminé dans lequel le groupe aminé de la chaîne latérale est substitué avec un acyle en C₁₋₅, un alkyle en C₁₋₅, un -B'-acyle en C₁₋₅ ou un -B'-alkyle en C₁₋₅, dans lequel B' est un résidu d'acide aminé qui peut éventuellement être substitué avec un ou plusieurs groupes nitro,
C - est Gly où le N-H peptidique peut éventuellement être modifié en N-méthyle, D - est Phe qui est substitué sur le cycle aromatique avec au moins un groupe nitro et éventuellement en outre substitué sur le cycle aromatique avec un ou plusieurs groupes, indépendamment choisis parmi un nitro, fluoro, chloro, bromo, iodo, CF₃ ou CN, dans lequel le N-H peptidique peut éventuellement être changé en N-méthyle,
et le C=O C-terminal du résidu d'acide aminé D est substitué avec un NH₂, NH-alkyle en C₁₋₅, NH-NH₂, NH-NH-alkyle en C₁₋₅ ou un O-alkyle en C₁₋₅ ; ou la terminaison C est le COOH ou le CH₂OH,
ou son sel pharmaceutiquement acceptable.

2. Composé selon la revendication 1, dans lequel le résidu d'acide diaminé en position B est dans une configuration D ou L.

3. Composé selon la revendication 1, dans lequel le NH₂ N-terminal est non substitué.

4. Composé selon l'une quelconque des revendications précédentes, dans lequel A - est choisi parmi Tyr, Thyronine, Phe ou Trp, le cycle aromatique pouvant éventuellement être substitué par un ou plusieurs groupes nitro, et de préférence A est Tyr non substitué.

5. Composé selon l'une quelconque des revendications précédentes, dans lequel B - est Dap, Dab, Orn, Lys, 4,5-déhydro-lysine ou de l'acide 2,6-diamino-4-hexynoïque, dans lequel le groupe amino de la chaîne latérale est substitué avec un acyle en C₁₋₅, un alkyle en C₁₋₅, un-B'-acyle en C₁₋₅ ou-B'-alkyle en C₁₋₅.

6. Composé selon l'une quelconque des revendications précédentes, dans lequel B - est Dab, Orn ou Lys, dans lequel le groupe amino de la chaîne latérale est substitué avec un acyle en C₁₋₅, un alkyle en C₁₋₅, un -B'-acyle en C₁₋₅ ou -B'-alkyle en C₁₋₅ et de préférence B est un Orn substitué avec un acyle en C₁₋₅.

7. Composé selon l'une quelconque des revendications précédentes, dans lequel le groupe amino de la chaîne latérale de B est substitué par un acyle en C₁₋₅ ou un -B'-acyle en C₁₋₅, de préférence un acyle en C₁₋₅, de préférence un acétyle.

8. Composé selon l'une quelconque des revendications précédentes, dans lequel B' - est Gly, Ala, Pro, Leu, Asn ou Met, de préférence Gly ou Asn.

9. Composé selon l'une quelconque des revendications précédentes, dans lequel C - est Gly non substitué.

10. Composé selon l'une quelconque des revendications précédentes, dans lequel D - est Phe substitué en position para avec un nitro et éventuellement en outre substitué sur le cycle aromatique avec un ou plusieurs groupes indépendamment choisis parmi un nitro, fluoro, chromo, bromo, iodo, CF₃ ou CN, dans lequel le N-H peptidique peut éventuellement être changé en N-méthyle, et de préférence le seul substituant sur le cycle aromatique est un para-nitro.

11. Composé selon l'une quelconque des revendications précédentes, dans lequel le C=O C-terminal du résidu d'acide aminé D est substitué avec un NH₂.

12. Composé selon la revendication 1, comprenant la structure suivante :
Tyr-D-Orn(Ac)-Gly-Phe(p-NO₂)-NH₂

13. Composé selon la revendication 1, choisi parmi les composés comprenant les structures suivantes :
Tyr-D-Lys(Ac)-Gly-Phe(p-NO₂)-NH₂ (2)
Tyr-D-Orn(Ac-Asn)-Gly-Phe(p-NO₂)-NH₂ (3)
Tyr-D-Dab(Ac)-Gly-Phe(p-NO₂)NH₂ (4)
Tyr-D-Orn(Ac-Gly)-Gly-Phe(p-NO₂)-NH₂ (5)

14. Composition pharmaceutique comprenant un composé selon l'une quelconque des revendications précédentes et un diluant, support ou excipient pharmacologiquement acceptable.

15. Composé selon l'une quelconque des revendications 1 à 13, ou composition pharmaceutique selon la revendication 14, pour utilisation comme médicament.

16. Utilisation d'un composé selon l'une quelconque des revendications 1 à 13, pour la fabrication d'un médicament pour un traitement protecteur des tissus, de préférence un traitement cardio-protecteur.

17. Utilisation d'un composé selon l'une quelconque des revendications 1 à 13, pour la fabrication d'un médicament pour un traitement anti-hypoxique.

18. Utilisation d'un composé selon l'une quelconque des revendications 1 à 13, pour la fabrication d'un médicament pour un traitement analgésique.

19. Utilisation d'un composé selon l'une quelconque des revendications 1 à 13, pour la fabrication d'un médicament pour un traitement anti-ischémique.

20. Utilisation d'un composé selon l'une quelconque des revendications 1 à 13, pour la fabrication d'un médicament pour un traitement protecteur des tissus, de préférence un traitement cardio-protecteur, en combinaison avec un traitement antihypoxique ou analgésique, ou les deux.

21. Composé selon l'une quelconque des revendications 1 à 13 pour utilisation dans un traitement protecteur des tissus, de préférence un traitement cardio-protecteur.

22. Composé selon l'une quelconque des revendications 1 à 13 pour utilisation dans un traitement anti-hypoxique.

23. Composé selon l'une quelconque des revendications 1 à 13 pour utilisation dans un traitement analgésique.

24. Composé selon l'une quelconque des revendications 1 à 13 pour utilisation dans un traitement anti-ischémique.

25. Composé selon l'une quelconque des revendications 1 à 13 pour utilisation dans un traitement protecteur des tissus, de préférence un traitement cardio-protecteur, en combinaison avec un traitement antihypoxique ou analgésique, ou les deux.
